(19) Europäisches Patentamt
European Patent Office
Office européen des brevets

(11) Publication number : **0 301 970 B1**

# (12) EUROPEAN PATENT SPECIFICATION

(45) Date of publication of patent specification : **21.11.91 Bulletin 91/47**

(51) Int. Cl.[5] : **A61K 47/10**, A61K 9/08, A61K 37/02, A61K 31/445, A61K 31/47

(21) Application number : **88401968.8**

(22) Date of filing : **28.07.88**

## (54) Method for dissolving arginineamides and pharmaceutical compositions containing them.

The file contains technical information submitted after the application was filed and not included in this specification

(30) Priority : **28.07.87 JP 188484/87**

(43) Date of publication of application : **01.02.89 Bulletin 89/05**

(45) Publication of the grant of the patent : **21.11.91 Bulletin 91/47**

(84) Designated Contracting States : **DE FR GB IT**

(56) References cited :
**US-A- 2 854 380**
**US-A- 4 258 192**
**GSTIRNER F.: "Einführung in die Verfahrenstechnik der Arzneiformung", Wissenschaftliche Verlagsges.mbH, Stuttgart, 1973**

(73) Proprietor : **MITSUBISHI KASEI CORPORATION**
**5-2, Marunouchi 2-chome Chiyoda-ku**
**Tokyo 100 (JP)**

(72) Inventor : **Ofuchi, Kunihiko 1-204**
**Mitsubishi-Kasei-Daini**
**Shataku 5- 9809-25 Doai-Honcho**
**Hasaki-machi Kashima-gun Ibaraki-ken (JP)**
Inventor : **Nomura, Tatsuo 1-303**
**Mitsubishi-Kasei-Daiichi**
**Shataku 4-9809-61 Doai-Honcho**
**Hasaki-machi Kashima-gun Ibaraki-ken (JP)**

(74) Representative : **Gutmann, Ernest et al**
**S.C. Ernest Gutmann - Yves Plasseraud 67, boulevard Haussmann**
**F-75008 Paris (FR)**

EP 0 301 970 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a method for dissolving $N^2$-arysulfonyl-L-arginineamides, hereafter referred to as "arginineamides", and more particularly argipidine, and pharmaceutical compositions containing them.

Arginineamides are known to have anti-thrombotic activities and are expected to be used as anti-thrombotic agents (please refer to Japanese Patent N° 1382377). However, it is very difficult to obtain a solution containing arginineamides at high concentration due to poor their solubility in water. Therefore these compounds are not suitable for the production of solutions at high concentration suitable for injection.

A method for producing arginineamides is disclosed in US patent 4,258,192. It comprises removing the $N^G$-substituent from an $N^G$-substitued-$N^2$-quinolylsufonyl-L-argininamide by means of hydrogenating the quinolyl moiety to the corresponding 1,2,3,4-tetrahydroquinolyl moiety, in the presence of an hydrogen-activing catalyst.

The $N^2$-arylsufonyl-L-argininamides are isolated by filtration of the catalyst followed by evaporation of the solvent, and then purified by trituration or recrystallization from a suitable solvent, such as diethyl ethertetrahydrofuran, diethyl ether-methanol and water-methanol, or may be chromographed on silica gel or alumina.

US patent 2,854,380 discloses a dosage form of reserpine with a vehicle having a stabilizing effect on the reserpine chemically and which also enhances the physiological activity of reserpine. A vehicle, presented as a most preferred one, is a combination of propylene glycol, sorbitol and ethanol in water. The use of sorbitol in the vehicle increases chemical stability and therapeutic effect. The sorbitol also improves the taste of the preparation in an oral dosage form and eliminates the need for preservatives since it sweetens the preparation without providing a fermentation medium for contaminating organisms. Both propylene glycol and ethanol act as solvents for reserpine which is practically insoluble in aqueous media and not only contribute to the chemical stability of the reserpine but also bring about a more rapid onset of physiological effect.

An article titled : "Einführung in die Verfahrenstechnik der Arzneiformung" (Introduction to the processing techniques in the production of drugs) by Dr. Fritz Gstirner, 1973 edited by the "Wissenschaftliche Verlagsgesellschaft MBH, Stuttgart, pages 271-272 discloses a process for improving the solubility of a drug principles which include both a hydrophobic and hydrophilic parts, by improving the capacity of said hydrophobic parts of being hydrated (hydrotropia) by "hydrotropic" additives, e.g. monohydric or polyhydric alcohols, such as ethanol, benzylic alcohol, glycols (propylenglycol, butylenglycol) glycerin, as well as esters and ethers of glycols and amides.

The invention provides a method for dissolving arginineamide comprising dissolving $N^2$-arylsulfonyl-L-arginineamide having the general formula (I)

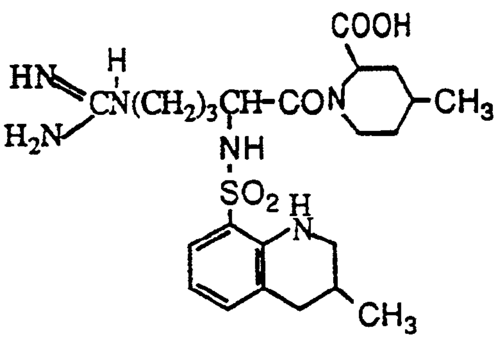

(I)

The invention can use the salts of the arginineamide having the general formula (I). The salts may be acid addition salts prepared by reacting with any inorganic or organic acids such as hydrochloric acid, hydrobromic acid, hydroiodic acid, sulfuric acid, nitric acid, phosphoric acid, acetic acid, citric acid, maleic acid, succinic acid, lactic acid, tartaric acid, gluconic acid, benzoic acid, methanesulfonic acid, ethanesulfonic acid, benzenesulfonic acid and p-toluenesulfonic acid. Further, the salts may be inorganic or organic salts prepared by reacting organic or inorganic bases such as sodium hydroxide, potassium hydroxide, ammonium hydroxide, triethylamine, procaine, dibenzylamine, N, N'-dibenzylethylenediamine and N-ethylpiperidine.

In the method for dissolving an arginineamide according to the invention, the arginineamide and/or its salt is dissolved in a solvent of ethanol and water. A mixture of ethanol and glycerin can be used.

Water used in the invention is generally distilled water or purified water, but a physiological saline or Ringer's solution may be used.

The mixed ratio (by weight) of alcohol to water in the above solvent is generally 0.1 to 10, preferably 0.2 to 5 and more preferably 0.3 to 3.

Saccharides can be admixed with the solvent of alcohol and water in the invention. As saccharides used in the invention, monosaccharides, oligosaccharides, polysaccharides and their reduced derivatives (for example sugaralcohol) which are soluble in water are mentioned. Among them, glucose, fructose, maltose, saccharose and D-sorbitol each of which has a high solubility in water are preferable. D-sorbitol is particularly preferable. A mixture of these saccharides may be used.

The mixed ratio (by weight) of saccharide (if present) to water is generally 0.1 to 10, preferably 0.4 to 4 and more preferably 0.5 to 2.

The manner how to dissolve the arginineamide having the general formula (I) in the solvent of alcohol and water and optionally saccharide is not particularly limited. Generally, the saccharide is dissolved in water and then the alcohol is added thereto followed by mixing. Next, the arginineamide is slowly added while stirring until complete dissolution.

The temperature on dissolution is not particularly limited. When the saccharide is dissolved in water, however, it is preferable to warm water at 40 to 70°C for accelerating the dissolution rate.

It is necessary to take care for preventing the evaporation of ethanol, for example by cooling the solution to room temperature before the dissolution, or dissolving in a closed container.

The concentration of arginineamide in the solution can be selected within the wide range depending on the intended uses. According to the invention, the solution in which the arginineamide is dissolved at high concentration, for example from several times to several thousands times the solubility of arginineamide in water can be obtained.

The solution containing the arginineamide having the general formula (I) in the solvent of ethanol water and glycerin or saccharide thus obtained can constitute the pharmaceutical composition of the invention.

The pharmaceutical compositions of the invention are useful for treating thrombosis. Accordingly, the pharmaceutical compositions can be used as the anti-thrombotic agents.

The pharmaceutical composition of the invention may contain antiseptics, anti-oxidants, soothing agents and pH-controlling agents. And, if necessary any pharmaceutical ingredient(S) other than the arginineamide may be added to form a combined preparation.

The pharmaceutical composition of the invention can be as an injection. This injectable composition may prepared contain stabilisers, buffers preservatives and which are acceptable for the injection and may be added in addition to the above-mentioned ingredients. If desired, the injectable composition according to the invention is prepared containing the arginineamide at very high concentration, and is used by diluting with water, electrolyte, carbohydrate solution or Ringer's solution on application such as by infusion or dialysis.

Alternatively, the pharmaceutical composition of the invention is topically applicable as a solution for topical application, or is useful for the production of an ointment or a suppository. When the pharmaceutical composition is used as a solution for topical application, the solution prepared above can be used as it is. And, the ointment or the suppository of the invention may be prepared by dissolving the solution prepared above in a base.

## EXAMPLES

The invention will now be further described by the following, non-limiting examples.

### Example 1

(2R, 4R)-1-[$N^2$-(3-methyl-1,2,3,4-tetrahydro-8-quinolinesulfonyl)-L-arginyl]-4-methyl-2-piperidinecarboxylic acid (argipidine) was dissolved in a solvent of ethanol and water while varying the mixed ratio of ethanol to water at 20°C (●) or 5°C (○).

This results are shown in Fig. 1. In Fig. 1, the ordinate is the solubility of argipidine in the solvent (mg/ml) and the abscissa is the weight percentages of ethanol and water (w/w %).

As shown in Fig. 1, the solubility of argipidine in the solvent comprising 70% by weight of ethanol and 30% by weight of water at 20°C was 33.23 mg/ml ant that at 5°C was 10.73 mg/ml.

### Comparative example 1

Argipidine was dissolved in the aqueous sorbitol solution while varying the sorbitol concentration at 20°C.

The result was shown in Fig. 2. In Fig. 2, the ordinate is the solubility of argipidine in the aqueous sorbitol solution (mg/ml) and the abscissa is the concentration of sorbitol in the aqueous solution (w/w %).

As shown in Fig. 2, the solubility of argipidine in the aqueous sorbitol solution was low and it was the substantially same as the solubility of argipidine in water.

Example 2

Argipidine was dissolved in a solvent comprising an aqueous 25% sorbitol solution (○), an aqueous 50% sorbitol solution (□) or a 70% sorbitol solution (△) and ethanol while varying the mixed ratio of ethanol to the aqueous sorbitol solution at 30°C.

The results are shown in Fig. 3. In Fig. 3, the ordinate is the solubility of argipidine in the solvent (mg/ml) and the abscissa is the weight percentages of the aqueous sorbitol solution and ethanol (w/w %).

Example 3

Argipidine was dissolved in a solvent comprising an aqueous 33% glucose solution (○), an aqueous 50% glycerine solution (△), a 50% sorbitol solution (●) or an aqueous 50% sucrose solution (□) and ethanol while varying the mixed ratio of ethanol to the aqueous solution at 30°C.

The results are shown in Fig. 4. In Fig. 4, the ordinate is the solubility of argipidine in the solvent (mg/ml) and the abscissa is the weight percentages of the aqueous solution and ethanol (w/w %).

Example 4

The distilled water for injection (200 g) was placed in a one-litre beaker, to which D-sorbitol (300 g) was added with stirring and dissolved. At this time, if necessary the solution may be heated. Then, ethanol (400 g) was added and mixed with stirring followed by adding argipidine (100 g) with stirring until complete dissolution.

The thus-obtained solution can be used for dialysis after diluting it with the weak acidic solution containing D-sorbitol.

Example 5

The distilled water for injection (200 g) was placed in a one-litre beaker, to which glucose (200 g) was added with stirring and dissolved. Then, ethanol (400 g) was added and mixed with stirring followed by adding argipidine (100 g) with stirring. Further, the distilled water for injection was added till the total volume of the solution became 1 litre.

The thus-obtained solution can be used for drip infusion after diluting it with the aqueous sorbitol solution, the aqueous D-sorbitol solution or Ringer's solution on use.

Example 6

The distilled water for injection (200 g) was placed in a one-litre beaker, to which -sorbitol (300 g) was added with stirring and dissolved. At this time, if necessary the solution may be heated. Then, glycerine (200 g) and ethanol (200 g) were added and mixed with stirring followed by adding argipidine (100 g) with stirring. Further, the distilled water for injection was added till the total volume of the solution became 1 litre.

The thus-obtained solution can be used for dialyses after diluting the weak acidic solution containing D-sorbitol.

EFFECT OF THE INVENTION

According to the method for dissolving the arginineamide of the invention, the injection containing an arginineamide having the general formula (I) and/or their salts, particularly at high concentration can be obtained.

## Claims

1. A method for dissolving an arginineamide comprising dissolving $N^2$-arylsulfonyl-L-arginineamide represented by the formula (I) :

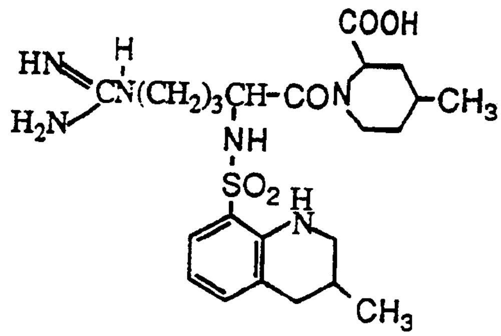

(I)

or a salt thereof or both in a solvent containing ethanol, water and a third component consisting of glycerin and or a saccharide.

2. The method according to claim 1, wherein the third component is a saccharide and the saccharide is at least one selected from sorbitol, glucose, maltose, fructose and sucrose.

3. The method according to claim 2, wherein the saccharide is D-sorbitol.

4. The method according to claim 1, wherein the mixed ratio of ethanol to water in the solvent is 0.1 to 10.

5. The method according to claim 4, wherein the mixed ratio of ethanol to water in the solvent is 0.2 to 5.

6. The method according to claim 5, wherein the mixed ratio of ethanol to water in the solvent is 0.3 to 3.

7. The method according to claim 1 alone or in combination with any of claims 2 to 6, wherein the mixed ratio of saccharide to water is 0.1 to 10.

8. The method according to claim 7, wherein the mixed ratio of saccharide to water is 0.4 to 4.

9. The method according to claim 8, wherein the mixed ratio of saccharide to water is 0.5 to 2.

10. A pharmaceutical composition for injection comprising $N^2$-arylsulfonyl-L-arginineamide represented by the formula (I) :

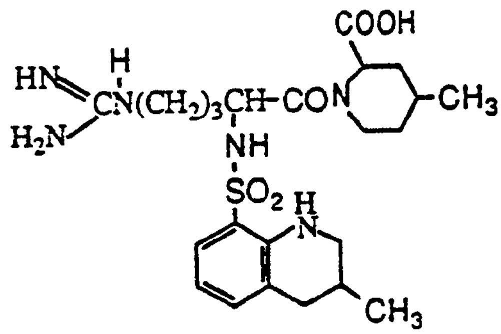

(I)

or a salt thereof or both in solubilized form in a mixture of ethanol, water and a third component consisting of glycerin and or a saccharide, as defined.

11. The composition according to claim 10, wherein the third component is saccharide and said saccharide is sorbitol, glucose, maltose, fructose or sucrose.

12. The composition according to claim 11, wherein the third component is a saccharide and said saccharide is D-sorbitol.

13. The composition of claim 10, wherein said third component is glycerin.

**Patentansprüche**

1. Verfahren zum Lösen von Argininamid, das das Lösen von $N^2$-Arylsulfonyl-L-argininamid, dargestellt durch die Formel (I)

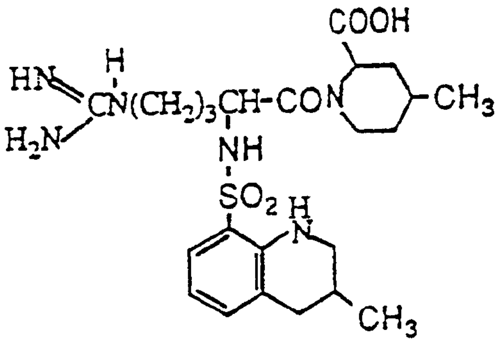

(I)

oder eines Salzes davon oder von beiden in einem Lösungsmittel, das Ethanol, Wasser und eine dritte Komponente, bestehend aus Glyzerin und/oder einem Saccharid, enthält, umfasst.

2. Verfahren nach Anspruch 1, wobei die dritte Komponente ein Saccharid ist, und das Saccharid mindestens eine Verbindung, ausgewählt aus Sorbit, Glukose, Maltose, Fructose und Sucrose ist.

3. Verfahren nach Anspruch 2, wobei das Saccharid D-Sorbit ist.

4. Verfahren nach Anspruch 1, wobei das Mischverhältnis von Ethanol zu Wasser in dem Lösungsmittel 0,1 bis 10 beträgt.

5. Verfahren nach Anspruch 4, wobei das Mischverhältnis von Ethanol zu Wasser in dem Lösungsmittel 0,2 bis 5 beträgt.

6. Verfahren nach Anspruch 5, wobei das Mischverhältnis von Ethanol zu Wasser in dem Lösungsmittel 0,3 bis 3 beträgt.

7. Verfahren nach Anspruch 1 allein oder in Kombination mit einem der Ansprüche 2 bis 6, wobei das Mischverhältnis von Saccharid zu Wasser 0,1 bis 10 ist.

8. Verfahren nach Anspruch 7, wobei das Mischverhältnis von Saccharid zu Wasser 0,4 bis 4 ist.

9. Verfahren nach Anspruch 8, wobei das Mischverhältnis von Saccharid zu Wasser 0,5 bis 2 ist.

10. Pharmazeutische Zusammensetzung zur Injekion, umfassend $N^2$-Arylsulfonyl-L-argininamid, dargestellt durch die Formel (I)

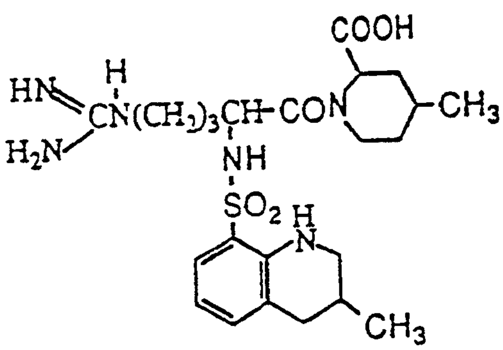

(I)

oder ein Salz hiervon oder beides in gelöster Form in einer Mischung aus Ethanol, Wasser und einer dritten Komponente, bestehend aus Glyzerin und/oder einem Saccharid.

11. Zusammensetzung nach Anspruch 10, wobei die dritte Komponente ein Saccharid ist, und das Saccharid Sorbit, Glukose, Maltose, Fructose oder Sucrose ist.

12. Zusammensetzung nach Anspruch 11, wobei die dritte Komponente ein Saccharid ist, und das Saccharid D-Sorbit ist.

13. Zusammensetzung nach Anspruch 10, wobei die dritte Komponente Glyzerin ist.

**Revendications**

1. Procédé pour dissoudre un argininamide comprenant la dissolution d'un $N^2$-arylsulfonyl-L-argininamide représenté par la formule (I) :

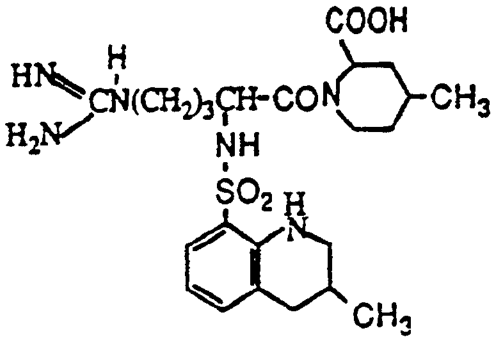

(I)

et/ou un sel de celui-ci, dans un solvant contenant de l'éthanol, de l'eau et un troisième composant constitué de glycérine et/ou d'un saccharide.

2. Procédé selon la revendication 1, dans lequel le troisième composant est un saccharide et le saccharide est au moins l'un de ceux choisis parmi le sorbitol, le glucose, le maltose, le fructose et le saccharose.

3. Procédé selon la revendication 2, dans lequel le saccharide est le D-sorbitol.

4. Procédé selon la revendication 1, dans lequel le rapport de mélange de l'éthanol à l'eau dans le solvant est de 0,1 à 10.

5. Procédé selon la revendication 4, dans lequel le rapport de mélange de l'éthanol à l'eau dans le solvant est de 0,2 à 5.

6. Procédé selon la revendication 5, dans lequel le rapport de mélange de l'éthanol à l'eau dans le solvant est de 0,3 à 3.

7. Procédé selon la revendication 1 seule ou en combinaison avec l'une quelconque des revendications 2 à 6, dans lequel le rapport de mélange du saccharide à l'eau est de 0,1 à 10.

8. Procédé selon la revendication 7, dans lequel le rapport de mélange du saccharide à l'eau est de 0,4 à 4.

9. Procédé selon la revendication 8, dans lequel le rapport de mélange du saccharide à l'eau est de 0,5 à 2.

10. Composition pharmaceutique pour l'injection comprenant un $N^2$-arylsulfonyl-L-argininamide représenté par la formule (I) :

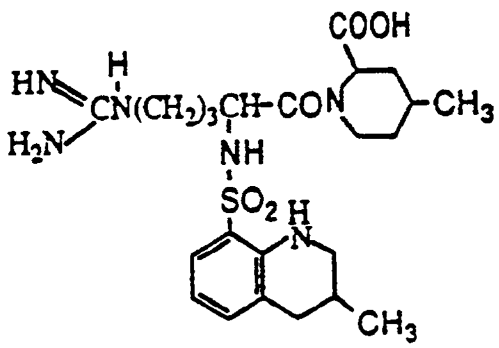

(I)

et/ou un sel de celui-ci, sous une forme solubilisée dans un mélange d'éthanol, d'eau et d'un troisième composant constitué de glycérine et/ou d'un saccharide,

11. Composition selon la revendication 10, dans laquelle le troisième composant est un saccharide et ledit saccharide est le sorbitol, le glucose, le maltose, le fructose ou le saccharose.

12. Composition selon la revendication 11, dans laquelle le troisième composant est un saccharide et ledit saccharide est le D-sorbitol.

13. Composition selon la revendication 10, dans laquelle le troisième composant est la glycérine.

Fig. 1

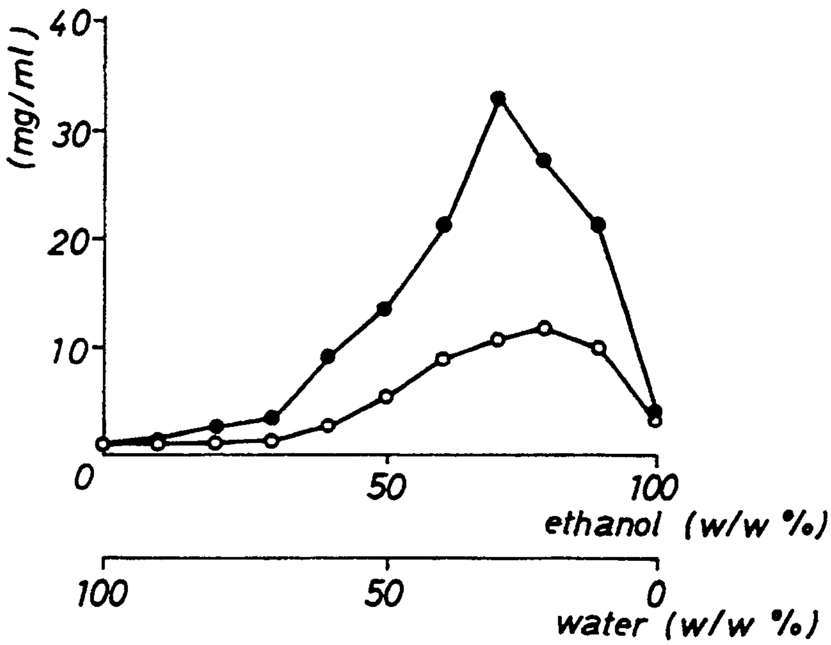
(mg/ml)
40
30
20
10
0
50
100
ethanol (w/w %)
100
50
0
water (w/w %)

Fig. 2

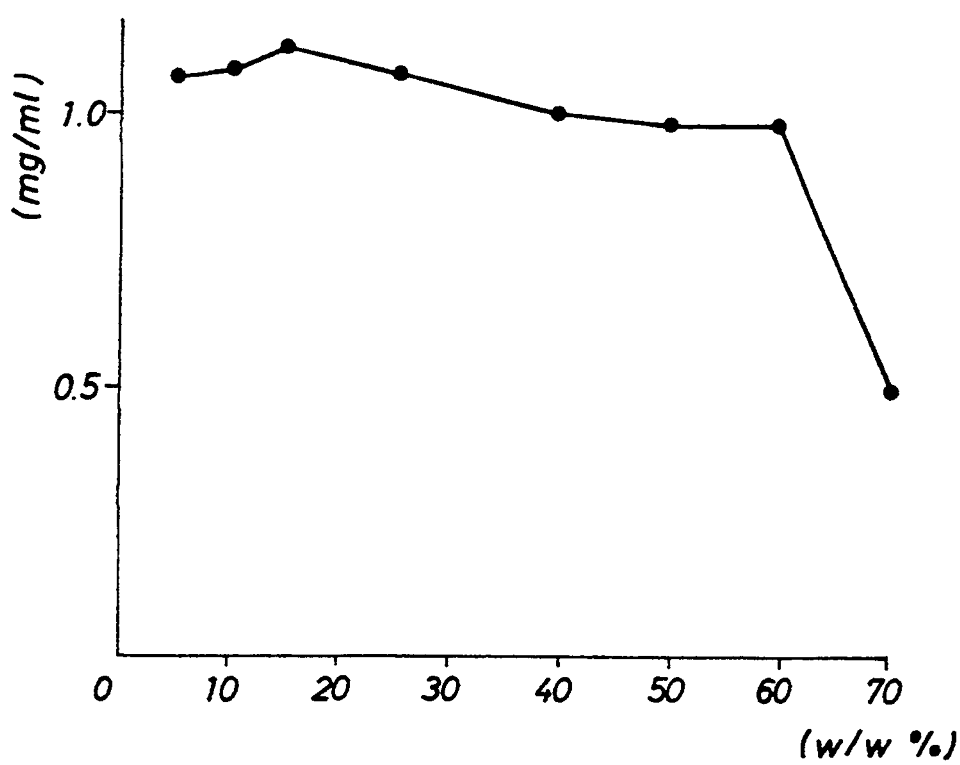
(mg/ml)
1.0
0.5
0
10
20
30
40
50
60
70
(w/w %)

Fig. 3

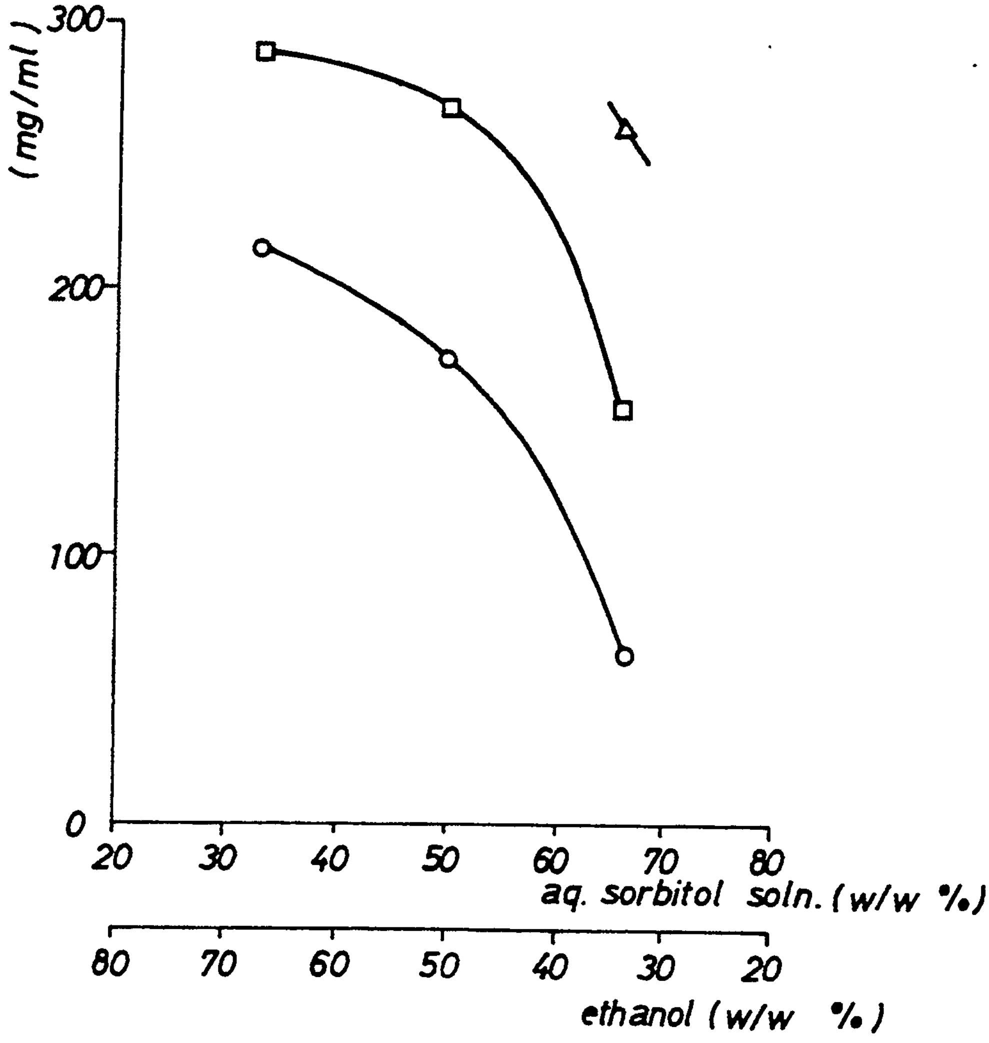
(mg/ml)
300
200
100
0
20
30
40
50
60
70
80
aq. sorbitol soln. (w/w %)
80
70
60
50
40
30
20
ethanol (w/w %)

Fig.4

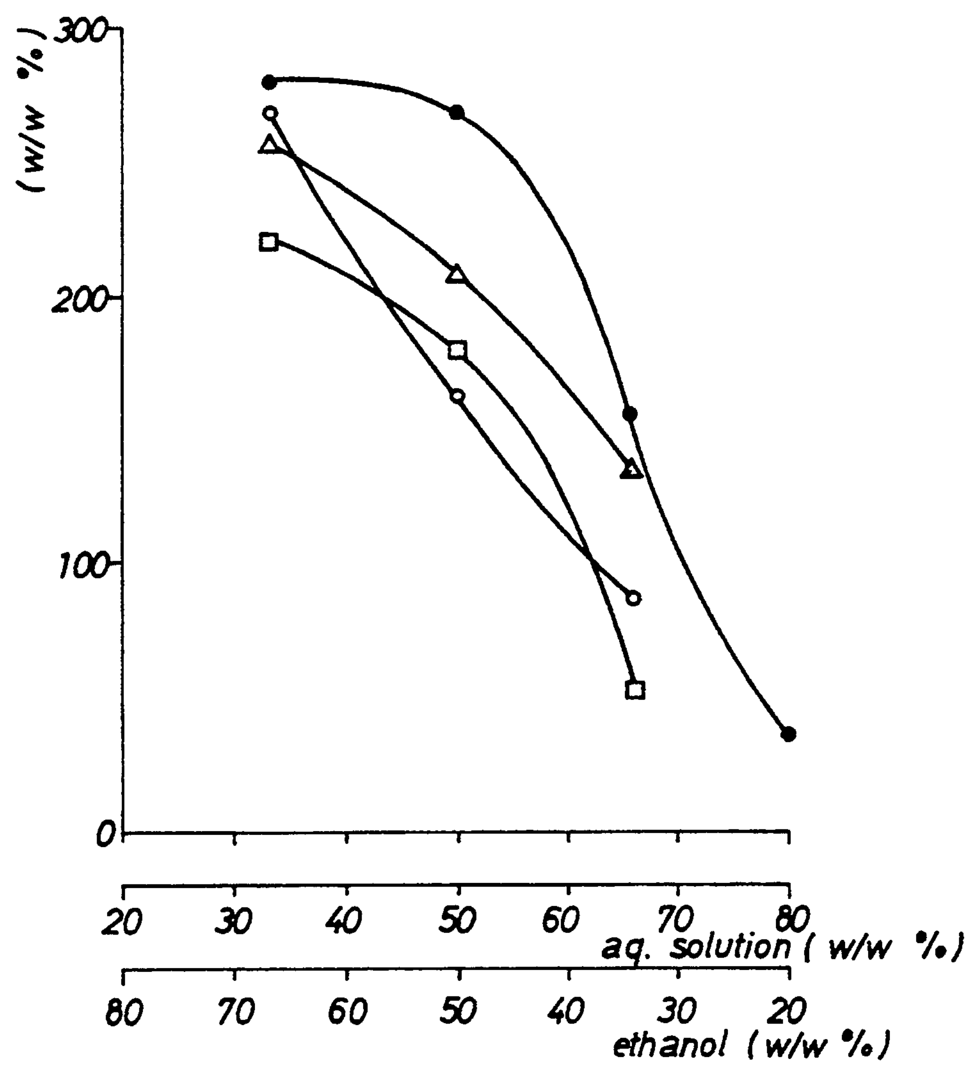
(w/w %)
300
200
100
0
20
30
40
50
60
70
80
aq. solution (w/w %)
80
70
60
50
40
30
20
ethanol (w/w %)